# FASCICULE DE BREVET EUROPEEN

(11) **EP 2 013 164 B1**
(45) Date de publication et mention de la délivrance du brevet: **02.11.2016**
(21) Numéro de dépôt: 07731313.8
(22) Date de dépôt: 18.04.2007
(51) Int. Cl.: C07C 313/04

(54) **PROCEDE DE PREPARATION DE DERIVES ORGANIQUES OXYSULFURES ET FLUORES**
VERFAHREN ZUR HERSTELLUNG VON OXISULFID UND FLUORIERTEN ORGANISCHEN DERIVATEN
METHOD FOR PREPARATION OF OXYSULFIDE AND FLUORINATED ORGANIC DERIVATIVES

(30) Priorité: 26.04.2006 FR 0603715
(43) Date de publication de la demande: 14.01.2009
(73) Titulaire: Rhodia Opérations, 75009 Paris (FR)
(72) Inventeur: BESSON, Bernard, 01700 Les Echets (FR); METZ, François, F-69540 Irigny (FR)
(74) Mandataire: Dutruc-Rosset, Marie-Claude
(86) Numéro de dépôt international: PCT/FR2007/000649
(87) Numéro de publication internationale: WO 2007/128893

(56) Documents cités:
- EP-A1- 0 735 023

## Description

La présente invention a pour objet un procédé perfectionné de préparation de dérivés organiques oxysulfurés et fluorés.

Plus précisément, l'invention concerne la préparation d'acides fluoroalcanesulfiniques et sulfoniques et de leurs sels.

L'invention vise la préparation d'acides perfluoroalcanesulfinique et sulfonique, et préférentiellement des acides trifluorométhanesulfinique et trifluorométhanesulfonique sous forme salifiée.

Les acides perhalogénoalcanesulfoniques et plus particulièrement l'acide trifluorométhanesulfonique sont utilisés comme catalyseurs ou comme intermédiaires en synthèse organique.

Dans EP 0 735 023, on a décrit la synthèse de dérivés organiques oxysulfurés et fluorés, notamment de l'acide perfluorométhanesuifinique sous forme salifiée, par réaction dans un solvant organique polaire, d'un acide fluorocarboxylique de formule Ea - CF₂ - COOH, où Ea représente un atome ou un groupe électro-attracteur, au moins partiellement salifié par un cation organique ou minéral et d'un oxyde de soufre, notamment du dioxyde de soufre et chauffage du mélange résultant à une température comprise entre 100°C et 200°C pendant une durée comprise entre 30 min et 20 heures.

Les quantités relatives dudit acide fluorocarboxylique initial et d'oxyde de soufre, de préférence dioxyde sont telles que le rapport entre le nombre d'atomes de soufre par mole d'acide fluorocarboxylique soit compris entre 1 et 10, avantageusement aux alentours de deux.

Selon EP 0 735 023, deux conditions apparaissent essentielles à savoir le choix du solvant et la teneur en protons libérables dans le mélange réactionnel.

Les rendements obtenus en dérivés organiques oxysulfurés et fluorés s'échelonnent entre 30 et 55 % avec une sélectivité de réaction de l'ordre de 50 % à l'exception de l'exemple 6 où une sélectivité de 85 % est obtenue grâce à la mise en oeuvre de conditions anhydres très poussées ce qui est très contraignant d'un point de vue industrielle.

De plus, il est à noter que pour éviter une dégradation trop importante du produit final, et donc assurer une bonne sélectivité de la réaction, il est préférable de ne pas chercher à convertir complètement l'acide fluorocarboxylique de départ. Ainsi, la réaction est conduite jusqu'à l'obtention d'un taux de transformation de 40 à 80 %, de préférence de 50 à 70 %.

Poursuivant ses recherches, la demanderesse a trouvé que ce procédé pouvait être perfectionné si l'on conduisait la réaction de sulfination, en limitant la quantité d'oxyde de soufre introduite.

Plus précisément, l'objet de la présente invention est un procédé de préparation d'un dérivé organique oxysulfuré et fluoré comprenant la mise en réaction, en présence d'un solvant organique polaire aprotique :
- (i) d'un acide fluorocarboxylique de formule Ea - CF₂ - COOH (I),
   où Ea représente un atome ou un groupe électro-attracteur, au moins partiellement salifié par un cation organique ou minéral,
- (ii) et de dioxyde de soufre, ledit procédé étant caractérisé par le fait que le rapport entre le nombre de moles de dioxyde de soufre et le nombre de moles d'acide fluorocarboxylique est inférieur à 1, de préférence inférieur à 0,99.

Conformément au procédé de l'invention, il a été trouvé que la sélectivité de la réaction de sulfination pouvait être améliorée dès lors que la quantité de dioxyde de soufre était inférieure à la quantité stoechiométrique. Ainsi, le rapport précédemment défini est choisi inférieur à 1, de préférence inférieur à 0,99 et plus préférentiellement compris entre 0,4 et 0,95.

Généralement, dans de telles réactions, les quantités d'oxyde de soufre sont en grands excès stoechiométriques et il n'était pas du tout évident pour l'Homme du Métier que la diminution dudit rapport permette d'obtenir les avantages apportés par l'invention.

Non seulement, la sélectivité de la réaction est améliorée mais également le procédé est mis en oeuvre plus facilement du fait que la pression de la réaction peut être abaissée et devenir égale à la pression atmosphérique.

Conformément au procédé de l'invention, on fait réagir un acide fluorocarboxylique sous forme salifiée avec du dioxyde de soufre.

Dans l'acide fluorocarboxylique répondant à la formule (I), l'entité Ea qui exerce un effet électro-attracteur sur l'atome de carbone difluoré est de préférence choisie parmi les groupes fonctionnels dont la constante de Hammett σₚ est au moins égale à 0,1.

Il est en outre préférable que la composante inductive de σₚ, σᵢ, soit au moins égale à 0,2, avantageusement à 0,3.

A cet égard, on se référera à l'ouvrage de Jerry MARCH - Advanced Organic Chemistry, 4ème édition, John Wiley and Sons, 1992, chapitre 9, pp. 278 - 286, et notamment au tableau 9.4 de cette section.

Plus particulièrement, l'entité électro-attractrice Ea est un atome de fluor.

L'acide fluorocarboxylique correspondant est un acide halogénofluoroacétique de formule (la) :

X-CF₂-COOH (la)

dans ladite formule :
- X est un atome de fluor.

Ea peut également être avantageusement choisie parmi les groupements carbonylés, sulfonés et perfluoroalkylés.

Des acides fluorocarboxyliques de ce type qui peuvent être utilisés répondent à la formule (Ib)

R-G-CF₂-COOH (Ib)

dans ladite formule :
- G représente un groupe fonctionnel C = O, S = O,
- G représente un groupe perfluoroalkylène -(CF₂)ₙ- où n est supérieur ou égal à 1,
- R représente un atome d'halogène, de préférence chlore ou fluor,
- R représente un résidu organique ou minéral indifférent, de préférence un radical organique tel que aryle, alkyle, ou aralkyle, éventuellement substitué,
- R peut également représenter un support solide minéral ou organique, tel qu'une résine.

On donne ci-après les significations préférées des radicaux organiques.

On entend par « alkyle », une chaîne hydrocarbonée linéaire ou ramifiée en C₁-C₁₅, de préférence en C₁-C₁₀ et encore plus préférentiellement en C₁-C₄. Des exemples de groupes alkyle préférés sont notamment méthyle, éthyle, propyle, isopropyle, butyle, isobutyle, t-butyle.

Par « cycloalkyle », on entend un groupe hydrocarboné cyclique, monocyclique en C₃-C₈, de préférence, un groupe cyclopentyle ou cyclohexyle ou polycyclique (bi- ou tricyclique) en C₄-C₁₈, notamment adamantyle ou norbornyle.

Par « aryle », on entend un groupe mono- ou polycyclique aromatique, de préférence, mono- ou bicyclique en C₆-C₂₀, de préférence, phényle ou naphtyle. Lorsque le groupe est polycyclique c'est-à-dire qu'il comprend plus d'un noyau cyclique, les noyaux cycliques peuvent être condensés deux à deux ou rattachés deux à deux par des liaisons σ. Des exemples de groupes (C₆-C₁₈)aryle sont notamment phényle, naphtyle.

Par « arylalkyle », on entend un groupe hydrocarboné, linéaire ou ramifié porteur d'un cycle aromatique monocyclique en C₇-C₁₂, de préférence, benzyle : la chaîne aliphatique comprenant 1 ou 2 atomes de carbone.

Il est à noter que dès lors que l'un des groupes comprend un cycle, celui-ci peut être substitué par un ou plusieurs, de préférence deux substituants. La nature du substituant peut être quelconque dès lors qu'il n'interfère pas au niveau de la réaction. Comme exemples préférés, on peut citer notamment les groupes alkyle ou alkoxy ayant de 1 à 4 atomes de carbone.

Dans le cadre de l'invention, les groupes alkyle sont les radicaux organiques préférés.

Dans le cas où G représente un groupe perfluoroalkylène -(CF₂)ₙ-, n est avantageusement compris entre 1 et 10, de préférence entre 1 et 5. Toujours dans ce cas, R peut également représenter un atome d'halogène, notamment le fluor.

De manière générale, sauf dans le cas où l'acide fluorocarboxylique est un polymère, le nombre total d'atomes de carbone de l'acide fluorocarboxylique n'excède avantageusement pas 30. Il est de préférence compris entre 2 et 12.

Les contre-cations susceptibles de former un sel avec ledit acide fluorocarboxylique sont avantageusement volumineux. Ainsi, on préfère des sels alcalins, avantageusement où ledit métal est choisi parmi le sodium, le potassium, le rubidium et le césium.

De préférence, le métal est d'une période dont le rang est au supérieur à celui du sodium, en particulier potassium ou césium.

Il est également possible d'améliorer la réaction en utilisant des cations qui sont, soit naturellement volumineux comme les cations ammonium quaternaire ou phosphonium quaternaire, ou rendus volumineux par l'addition d'agents chélatants ou de préférence cryptands, tels que par exemple les éthers couronne ou les dérivés qui sont à la fois aminés et oxygénés.

Comme cations ammonium ou phosphonium quaternaire, on met en oeuvre préférentiellement, les tétralkylammonium ou phosphonium, trialkylbenzylammonium ou phosphonium, tétraarylammonium ou phosphonium dont les groupes alkyle identiques ou différents représentent une chaîne alkyle linéaire ou ramifiée ayant de 4 à 12 atomes de carbone, de préférence de 4 à 6 atomes de carbone et le groupe aryle est avantageusement un groupe phényle.

On choisit préférentiellement le cation tétrabutylammonium.

Des sels d'acides perfluorocarboxyliques peuvent être avantageusement utilisés, tels que les trifluoroacétate, perfluoropropionate, perfluorobutyrate, perfluorooctanoate de métal alcalin, notamment de potassium.

L'invention concerne préférentiellement la préparation de l'acide trifluorométhanesulfinique sous forme salifiée, de préférence sous forme d'un sel de métal alcalin et plus préférentiellement de potassium.

L'oxyde de soufre, qui est le dioxyde de soufre, peut être mis en oeuvre sous forme gazeuse. Il peut être également introduit sous forme de solution, dans le solvant organique choisi pour la réaction, à une concentration variant généralement entre 1 et 10 % en poids, de préférence entre 3 et 6 %.

Selon la caractéristique du procédé de l'invention, le rapport entre le nombre de moles d'oxyde de soufre et le nombre de moles d'acide fluorocarboxylique est inférieur à 1, de préférence inférieur à 0,99.

Ledit rapport est choisi de préférence entre 0,4 et 0,95 et préférentiellement entre 0,7 et 0,9.

Le procédé de l'invention consiste à effectuer la réaction de sulfination dans un solvant organique.

Ce dernier joue un rôle important dans la présente invention et doit être aprotique, et avantageusement polaire et comporter très peu d'impuretés porteuses d'hydrogène acide.

On entend par solvant aprotique, un solvant qui, dans la théorie de Lewis n'a pas de protons à libérer.

On fait appel à un solvant suffisamment stable dans les conditions réactionnelles.

Il est souhaitable que le solvant solubilise le sel d'acide fluorocarboxylique soit au moins partiellement, de préférence complètement.

Ainsi, le solvant organique est choisi polaire. Il est ainsi préférable que le solvant aprotique polaire utilisable ait un moment dipolaire significatif. Ainsi, sa constante diélectrique relative ε est avantageusement au moins égale à environ 5. De préférence, sa constante diélectrique est inférieure ou égale à 50 et supérieure ou égale à 5, et est notamment comprise entre 30 et 40.

Afin de déterminer si le solvant organique répond aux conditions de constante diélectrique énoncées ci-dessus, on peut se reporter, entre autres, aux tables de l'ouvrage : Techniques of Chemistry; II - Organic solvents - p. 536 et suivantes, 3ème édition (1970).

On préfère en outre que les solvants de l'invention soient susceptibles de bien solvater les cations, ce qui signifie que le solvant présente certaines propriétés de basicité au sens de Lewis.

Pour déterminer si un solvant satisfait à cette exigence, on apprécie sa basicité en se référant au "nombre donneur". On choisit un solvant organique polaire présentant un nombre donneur supérieur à 10, de préférence supérieur ou égal à 20. La borne supérieure ne présente aucun caractère critique. On choisit, de préférence, un solvant organique ayant un nombre donneur compris entre 10 et 30.

On rappellera que le "nombre donneur" ou "donor number" désigné de manière abrégée DN, donne une indication sur le caractère nucléophile du solvant et révèle son aptitude à donner son doublet.

Dans l'ouvrage de Christian REICHARDT, [Solvents and Solvent Effects in Organic Chemistry - VCH p. 19 (1990)], on trouve la définition du "donor number" qui est défini comme le négatif (-ΔH) de l'enthalpie (Kcal/mol) de l'interaction entre le solvant et le pentachlorure d'antimoine, dans une solution diluée de dichloroéthane.

Selon la présente invention, le ou les solvants polaires ne présentent pas d'hydrogène acide, en particulier lorsque le caractère polaire du ou des solvants est obtenu par la présence de groupes électro-attracteurs, il est souhaitable qu'il n'y ait pas d'hydrogène sur l'atome en position α de la fonction électro-attractrice.

De manière plus générale, il est préférable que le pKa correspondant à la première acidité du solvant soit au moins égal à environ 20 ("environ" soulignant que seul le premier chiffre est significatif), avantageusement au moins égal à environ 25, de préférence compris entre 25 et 35.

Le caractère acide peut également être exprimé par l'indice accepteur AN du solvant, tel qu'il est défini par Christian REICHARDT, ["Solvents and solvent effects in Organic Chemistry", 2ème édition, VCH (RFA), 1990, pages 23-24].

Avantageusement, cet indice accepteur AN est inférieur à 20, en particulier inférieur à 18.

Les solvants répondant aux différents impératifs et donnant de bons résultats peuvent être notamment des solvants de type amide. Parmi les amides, on comprend aussi les amides à caractère particulier comme les urées tétrasubstituées et les lactames monosubstitués. Les amides sont de préférence substitués (disubstitués pour les amides ordinaires). On peut citer par exemple les amides tels que le N,N-diméthylformamide (DMF), le N,N-diéthylformamide ou encore le N,N-diméthylacétamide ; les dérivés de pyrrolidone, tels que la N-méthylpyrrolidone,

Une autre catégorie particulièrement intéressante de solvant est constituée par les éthers, qu'ils soient symétriques ou non symétriques, qu'ils soient ouverts ou non. Dans la catégorie des éthers doivent être incorporés les différents dérivés des éthers de glycol tels que les différents glymes, le diglyme par exemple.

Parmi les solvants précités, on met en oeuvre, préférentiellement, le DMF.

La quantité de solvant organique à mettre en oeuvre est déterminée en fonction de la nature du solvant organique choisi.

Elle est déterminée de telle sorte que la concentration de l'acide fluorocarboxylique dans le solvant organique soit de préférence comprise entre 1 et 30 % en poids et plus préférentiellement entre 10 et 20 %.

Selon des conditions préférées de mise en oeuvre du procédé de l'invention, il est souhaitable de contrôler la teneur en impuretés présente dans le milieu réactionnel.

La teneur en atomes d'hydrogène labiles du système, ou plus exactement en protons libérables portés par ses divers composants, y compris leurs impuretés, doit être inférieure à la teneur en groupes fluorés libérés par la décomposition des acides fluorocarboxyliques.

Par atome d'hydrogène labile ou proton libérable, on entend un atome d'hydrogène qui est susceptible d'être arraché sous forme de proton par une base forte. En pratique, il s'agit des protons des fonctions acides qui présentent un pKa inférieur à environ 20.

Plus la teneur en protons libérables sera faible, moins il y aura de risque de réaction parasite et meilleur sera le rendement.

La teneur en protons libérables présents dans le milieu est au plus égale à 20 % de la concentration molaire initiale dudit acide fluorocarboxylique.

Avantageusement cette teneur est au plus égale à 10 %, de préférence à 1 % (en moles), par rapport à la teneur initiale en ledit acide fluorocarboxylique.

La principale molécule porteuse d'atomes d'hydrogène labiles, est en général l'eau qui est susceptible de libérer jusqu'à deux protons par molécule.

D'une manière générale il est préférable d'utiliser des réactifs et des solvants déshydratés, de manière que la teneur pondérale en eau du réactif soit au plus égale à 1 pour 1000, par rapport à la masse totale du réactif.

En fonction de l'ensemble des conditions réactionnelles, de telles teneurs en eau peuvent être satisfaisantes, mais dans certains cas, il peut être intéressant d'opérer à des niveaux plus bas, par exemple de l'ordre de 1 pour 10 000.

Toutefois, il n'est pas nécessairement indispensable d'éliminer la totalité de l'eau et un rapport molaire eau/acide fluorocarboxylique inférieur à 10 % peut être toléré.

Comme mentionné dans EP 0 735 023, il est souhaitable que les impuretés métalliques soient en faibles quantités. Des éléments métalliques peuvent être présents en tant qu'impuretés apportées notamment par les réactifs, le solvant ou bien par les appareillages métalliques suite à une corrosion.

Ainsi, afin de ne pas apporter de pollution métallique additionnelle, il importe que le sel de l'acide fluorocarboxylique de départ mis en oeuvre soit préparé par réaction d'une base avec l'acide fluorocarboxylique de formule (I) dans des conditions telles que la base soit introduite en une quantité voisine et de préférence égale à la quantité stoechiométrique.

D'une manière plus générale, on peut indiquer que les deux catégories de métaux, à savoir les éléments de transition à deux états de valence (tels que le cuivre) et les éléments de la colonne VIII (notamment des métaux de la mine du platine qui est le groupe constitué du platine, de l'osmium, de l'iridium, du palladium, du rhodium et du ruthénium), doivent être présents dans le milieu à une teneur exprimée par rapport à l'acide fluorocarboxylique au plus égale à 1000 ppm molaires, de préférence à 10 ppm molaires.

Dans la présente description, il est fait référence au supplément au bulletin de la Société Chimique de France N°1, janvier 1966, où une classification périodique des éléments a été publiée.

Conformément au procédé de l'invention, on met en contact l'acide fluorocarboxylique sous forme salifiée, l'oxyde de soufre et le solvant organique.

La mise en oeuvre peut être effectuée de manière discontinue (en batch) ou d'une manière continue.

Les modes d'introduction ne sont pas critiques mais certains sont préférés.

Selon un mode de mise en oeuvre en discontinu, on peut introduire le sel de l'acide fluorocarboxylique dans le solvant organique puis ajouter en totalité ou par fractions l'oxyde de soufre : ce dernier peut être introduit sous forme gazeuse par absorption dans le milieu précité ou bien introduit également en solution dans un solvant organique, de préférence, celui de la réaction.

La réaction est conduite dans un réacteur classique équipé d'un dispositif de chauffage (échangeur thermique) et d'un dispositif d'agitation, par exemple, une agitation à l'aide d'une hélice.

Ensuite, on chauffe le mélange réactionnel.

Selon un mode de réalisation en continu, on fait appel à un appareillage permettant une mise en oeuvre en continu tel que plusieurs réacteurs en cascade ou un tube équipé d'une double enveloppe dans lequel circule un fluide caloporteur dont les caractéristiques permettent d'atteindre la température réactionnelle souhaitée.

Dans ce cas, on alimente le dispositif par le sel de l'acide fluorocarboxylique en mélange avec le solvant organique et l'on introduit le dioxyde de soufre,

Ce dernier peut être ajouté dans la solution d'alimentation comprenant l'acide fluorocarboxylique et le solvant organique ou bien il est peut être introduit à différents endroits de l'appareillage : l'arrivée pouvant être faite dans le ciel du réacteur ou dans la masse réactionnelle.

Ensuite, on effectue le chauffage jusqu'à obtention du taux de transformation souhaité.

Conformément au procédé de l'invention, le chauffage du mélange réactionnel a lieu avantageusement à une température comprise entre 100°C et 200°C, de préférence entre 120°C et 160°C.

La réaction de sulfination est avantageusement mise en oeuvre sous pression atmosphérique mais des pressions plus élevées peuvent être également utilisées. Ainsi, peut convenir une pression totale absolue choisie entre 1 et 20 Bar et de préférence entre 1 et 3 Bar

La durée du chauffage peut varier largement en fonction de la température de réaction choisie. Elle peut varier entre environ 30 min à au plus une journée. Elle est avantageusement de plus d'une heure à moins de 20 heures et plus préférentiellement comprise entre 2 heures et 7 heures.

Selon le mode de réalisation en continu, le temps de séjour moyen qui est défini comme le rapport entre le volume de la masse réactionnelle et le débit d'alimentation, se situe entre 30 min et 10 heures, et plus préférentiellement entre 2 heures et 4 heures.

Ledit oxyde de soufre étant du dioxyde de soufre, le mélange résultant de l'étape de sulfination peut comprendre deux phases : une phase liquide, où une partie au moins dudit acide et du dioxyde de soufre sont dissous dans ledit solvant et une phase gazeuse contenant essentiellement du dioxyde de soufre et du gaz carbonique formé au cours de la réaction.

Pour éviter une dégradation trop importante du produit final, et donc assurer une bonne sélectivité de la réaction, il est préférable de ne pas chercher à convertir complètement l'acide fluorocarboxylique de départ.

L'avancement de la réaction peut être contrôlé par le taux de conversion (TT) de l'acide qui est le rapport molaire de la quantité d'acide disparue sur la quantité d'acide initiale dans le milieu réactionnel, ce taux étant calculé aisément après dosage de l'acide restant dans le milieu.

De manière avantageuse, on ne conduira la réaction que jusqu'à l'obtention d'un taux de transformation de 30 à 80 %, de préférence de 40 à 60 %, puis on séparera les produits réactionnels. Il est possible d'atteindre ainsi une sélectivité supérieure à 80 %, voire supérieure à 90 % exprimée par le rapport molaire produit recherché/acide fluorocarboxylique transformé.

Une fois atteint le taux de transformation désiré, le mélange réactionnel peut être traité de façon connue en soi pour séparer le produit obtenu, les produits de départ pouvant être recyclés pour produire une quantité supplémentaire du dérivé organique visé.

Ledit oxyde de soufre étant le dioxyde de soufre, le produit obtenu par chauffage du milieu réactionnel est un sel d'acide sulfinique dont le contre-ion est celui du sel d'acide fluorocarboxylique de départ.

Pour séparer le produit de la réaction, une possibilité avantageuse consiste à procéder à une transformation supplémentaire en un dérivé relativement volatil et facilement distillable.

Ainsi, par exemple, au cours de la réaction entre SO₂ et les sels de l'acide trifluoroacétique CF₃CO₂H, les sels de l'acide trifluorométhylsulfinique CF₃SO₂H obtenus peuvent aisément être convertis en présence de chlore Cl₂ en CF₃SO₂Cl (cette réaction est générale aux acides utilisés et notamment aux acides perfluoroalcanesulfiniques R_{f} SO₂H). Cette réaction permet avantageusement de séparer CF₃SO₂Cl par distillation en laissant des chlorures minéraux ainsi que le sel de l'acide trifluorométhanesulfonique intact dans le milieu réactionnel, qui peut donc être réutilisé pour poursuivre la réaction avec l'oxyde de soufre. Cette réaction est commune aux différents acides sulfiniques fluorés que l'on peut obtenir selon l'invention. Cet exemple peut être généralisé à la séparation de tous types de dérivés organiques oxysulfurés fluorés obtenus selon l'invention susceptibles d'être transformés par une réaction appropriée en des produits plus volatils.

Pour passer de l'acide sulfinique à l'acide sulfonique correspondant, il convient de soumettre le produit réactionnel ou le produit de réaction purifié à une oxydation, en elle-même connue, notamment au moyen d'eau oxygénée ou d'hypochlorite de sodium. Un procédé de purification de trifluorométhylsulfinate de sodium, et d'oxydation en sulfonate, applicable selon l'invention est décrit dans la demande de brevet européen publiée sous le numéro EP-A-0 396 458.

Les sels d'acides sulfiniques ou sulfoniques ainsi obtenus peuvent être convertis en les acides libres correspondants en milieu acide. On utilise préférentiellement l'acide sulfurique éventuellement sous forme d'oléums ou bien l'acide chlorhydrique.

Les produits de réaction, sels ou acides libres, peuvent être isolés aisément et mis en oeuvre dans des étapes ultérieures de synthèse organique. Ainsi, par exemple, on peut valoriser les chlorures de sulfinyle obtenus à partir d'acides sulfiniques fluorés préparés selon l'invention.

Les exemples qui suivent, illustrent l'invention sans toutefois la limiter.

On donne ci-après la signification des abréviations utilisées dans les exemples.

Le taux de conversion (TT) correspond au rapport entre le nombre de moles de substrat transformées et le nombre de moles de substrat engagées.

Le rendement (RR) correspond au rapport entre le nombre de moles de produit formées et le nombre de moles de substrat engagées.

La sélectivité (RT) correspond au rapport entre le nombre de moles de produit formées et le nombre de moles de substrat transformées au cours de la réaction.

TFAK signifie « trifluoroacétate de potassium ».

Les exemples 1 et 2 donnés ci-après décrivent un mode de préparation en discontinu.

Les exemples 3 à 6 à illustrent un mode de réalisation en continu.

### Exemple 1 :

### Préparation de trifluorométhylsulfinate de potassium.

Dans un réacteur de 500 cm³ équipé d'une double enveloppe, d'une agitation mécanique centrale et d'une sortie sur l'atmosphère et d'un condenseur acétone/carbog lace permettant le reflux du dioxyde de soufre, on charge à température ambiante (environ 20°C), 125,5 g de diméthylformamide.

On introduit 25,5 g de trifluoroacétate de potassium dans le DMF.

On charge ensuite 6,9 g de dioxyde de soufre par l'intermédiaire d'un capillaire relié à une bouteille de dioxyde de soufre sous pression.

On chauffe à 140°C et sous pression atmosphérique.

Le rapport molaire de SO₂ par rapport au TFAK est de 0,64.

Au bout de 4 heures 25 min, l'analyse par chromatographie ionique donne les résultats suivants :

| | |
|---|---|
| - Taux de conversion du trifluoroacétate de potassium : | 57,1 % |
| - Rendement en trifluorométhylsulfinate de potassium : | 52,8 % |
| - Sélectivité en trifluorométhylsulfinate de potassium : | 92,4 % |

On note une très bonne sélectivité de réaction.

### Exemple 2 :

### Préparation de trifluorométhylsulfinate de potassium.

On reproduit l'exemple 1 à la différence près que le rapport molaire de SO₂ par rapport au TFAK est de 0,72.

Dans un appareillage tel que décrit dans l'exemple 1, on charge 116,2 g de diméthylformamide, 23,8 g de trifluoroacétate de potassium dans le DMF et l'on ajoute par bullage 7,2 g de dioxyde de soufre.

On chauffe à 137°C et sous pression atmosphérique.

Au bout de 5 heures, l'analyse par chromatographie ionique donne les résultats suivants :

| | |
|---|---|
| - Taux de conversion du trifluoroacétate de potassium : | 52 % |
| - Rendement en trifluorométhylsulfinate de potassium : | 47,4 % |
| - Sélectivité en trifluoroméfihylsulfinate de potassium : | 90,4 % |

On note une très bonne sélectivité de réaction.

### Exemples 3 à 6 :

### Préparation de trifluorométhylsulfinate de potassium.

On donne ci-après une série d'exemples selon une voie continue.

Dans les différents exemples qui suivent, on utilise un réacteur de forme cylindrique dont le chauffage est assuré par un fluide caloporteur (huile silicone) qui circule dans une double enveloppe.

Le réacteur est composé de quatre compartiments qui communiquent par des orifices situés au pied des parois les séparant.

Ainsi le liquide réactionnel qui est alimenté dans le premier compartiment à partir d'une nourrice et via une pompe volumétrique, peut passer dans le second compartiment et ainsi de suite jusqu'au dernier compartiment.

La masse réactionnelle sort par débordement du dernier compartiment et est recueilli dans un récipient par gravité.

Chaque compartiment est équipé d'un système d'agitation mécanique à arbre verticale, d'un système de respiration indépendant à pression atmosphérique et d'une sonde de température ainsi que d'une ouverture latérale permettant de prélever la phase liquide par l'intermédiaire d'une seringue.

Le volume utile c'est-à-dire le volume de masse réactionnelle de chaque compartiment est d'environ 50 cm³.

### Exemple 3 :

Le réacteur est alimenté à un débit de 1,3 cm³ .min⁻¹, par une solution ayant la composition suivante :

| | |
|---|---|
| - Diméthylformamide : | 82,1 % |
| - Trifluoroacétate de potassium : | 13,4 % |
| - Dioxyde de soufre : | 4,5 % |

Le rapport molaire de SO₂ par rapport au TFAK est de 0,8

Les quatre compartiments sont chauffés à 140°C.

Après un temps d'opération de 21 heures, l'état stationnaire est atteint.

La composition des différents compartiments est analysée par chromatographie ionique.

On trouve que dans chaque compartiment la conversion du TFAK, le rendement et la sélectivité en trifluorométhylsulfinate sont les suivants :

**Tableau (I)**

| N° compartiment | Conversion TFAK | Rendement en trifluorométhylsulfinate | Sélectivité en trifluorométhylsulfnate |
|---|---|---|---|
| 1 | 11,7 % | 11,3 % | 97,3 % |
| 2 | 24,4 % | 23,6 % | 96,8 % |
| 3 | 31,5 % | 31,3 % | 99,5 % |
| 4 | 37,0 % | 36,3 % | 98,2 % |

### Exemple 4 :

Le réacteur est alimenté à un débit de 0,7 cm³.min⁻¹, par une solution ayant la composition suivante :

| | |
|---|---|
| - Diméthylformamide : | 82,1 % |
| - Trifluoroacétate de potassium : | 13,4 % |
| - Dioxyde de soufre : | 4,5 % |

Le rapport molaire de SO₂ par rapport au TFAK est de 0,8.

Les quatre compartiments sont chauffés à 135°C.

Après un temps d'opération de 5 heures, on peut considérer que l'état stationnaire est atteint.

La composition des différents compartiments est analysée par chromatographie ionique.

On trouve que dans chaque compartiment la conversion du TFAK, le rendement et la sélectivité en trifluorométhylsulfinate sont les suivants:

**Tableau (II)**

| N° compartiment | Conversion TFAK | Rendement en trifluorométhylsulfinate | Sélectivité en trifluorométhylsulfinate |
|---|---|---|---|
| 1 | 21,2 % | 21,1 % | 99,1 % |
| 2 | 33,5 % | 32,2 % | 95,9 % |
| 3 | 38,0 % | 36,8 % | 97,1 % |
| 4 | 40,8 % | 38,0 % | 93,2 % |

### Exemple 5 :

Le réacteur est alimenté à un débit de 1,7 cm³.min⁻¹, par une solution ayant la composition suivante :

| | |
|---|---|
| - Diméthylformamide : | 78,5 % |
| - Trifluoroacétate de potassium : | 16,1 % |
| - Dioxyde de soufre : | 5,4 % |

Le rapport molaire de SO₂ par rapport au TFAK est de 0,8

Les quatre compartiments sont chauffés à 140°C.

Après un temps d'opération de 5 heures, on peut considérer que l'état stationnaire est atteint.

La composition des différents compartiments est analysée par chromatographie ionique.

On trouve que dans le quatrième compartiment la conversion du TFAK et le rendement en trifluorométhylsulfinate sont respectivement de 29,1 % et 28,1 %.

La sélectivité en trifluorométhylsulfiriate de potassium est de 96,4 %.

### Exemple 6 :

Le réacteur est alimenté à un débit de 1,7 cm³.min⁻¹, par une solution ayant la composition suivante :

| | |
|---|---|
| - Diméthylformamide : | 83,1 % |
| - Trifluoroacétate de potassium : | 13,5 % |
| - Dioxyde de soufre : | 3,4 % |

Le rapport molaire de SO₂ par rapport au TFAK est de 0,6.

Les quatre compartiments sont chauffés à 135°C.

Après un temps d'opération de 5 heures, on peut considérer que l'état stationnaire est atteint.

La composition des différents compartiments est analysée par chromatographie ionique.

On trouve que dans le quatrième compartiment la conversion du TFAK et le rendement en trifluorométhylsulfinate sont respectivement de 37 % et 35 %.

La sélectivité en trifluorométhylsulfinate de potassium est de 94 %.

## Revendications

1. Procédé de préparation d'un dérivé organique oxysulfuré et fluoré comprenant la mise en réaction, en présence d'un solvant organique polaire aprotique :
- (i) d'un acide fluorocarboxylique de formule Ea - CF₂ - COOH (I), où Ea représente un atome ou un groupe électro-attracteur, au moins partiellement salifié par un cation organique ou minéral,
- (ii) et de dioxyde de soufre,
ledit procédé étant **caractérisé par le fait que** le rapport entre le nombre de moles de dioxyde de soufre et le nombre de moles d'acide fluorocarboxylique est inférieur à 1, de préférence inférieur à 0,99.

2. Procédé selon la revendication 1 **caractérisé par le fait que** le rapport entre le nombre de moles de dioxyde de soufre et le nombre de moles d'acide fluorocarboxylique est compris entre 0,4 et 0,95, de préférence entre 0,7 et 0,9.

3. Procédé selon l'une des revendications 1 et 2 **caractérisé par le fait que** l'acide fluorocarboxylique répondant à la formule (I) dans laquelle l'entité Ea qui exerce un effet électro-attracteur sur l'atome de carbone difluoré est de préférence choisie parmi les groupes fonctionnels dont la constante de Hammett σₚ est au moins égale à 0,1.

4. Procédé selon l'une des revendications 1 à 3 **caractérisé par le fait que** l'acide fluorocarboxylique correspondant est un acide halogénofluoroacétique de formule (la) :
X-CF₂-COOH (la)
dans ladite formule :
- X est un atome de fluor.

5. Procédé selon l'une des revendications 1 à 3 **caractérisé par le fait que** l'acide fluorocarboxylique correspondant est un acide de formule (Ib) :
R-G-CF₂-COOH (Ib)
dans ladite formule :
- G représente un groupe fonctionnel C = O, S = O,
- G représente un groupe perfluoroalkylène -(CF₂)ₙ- où n est supérieur ou égal à 1,
- R représente un atome d'halogène, de préférence chlore ou fluor,
- R représente un résidu organique ou minéral indifférent, de préférence un radical organique tel que aryle en C₆-C₂₀, alkyle en C₁-C₁₅, ou aralkyle en C₇-C₁₂, éventuellement substitué,
- R peut également représenter un support solide minéral ou organique, tel qu'une résine.

6. Procédé selon la revendication 5 **caractérisé par le fait que** l'acide fluorocarboxylique correspondant est un acide halogénofluoroacétique de formule (Ib) dans laquelle G représente un groupe perfluoroalkylène -(CF₂)ₙ-, n étant avantageusement compris entre 1 et 10, de préférence entre 1 et 5.

7. Procédé selon l'une des revendications 1 à 6 **caractérisé par le fait que** ledit acide est salifié par un cation de métal alcalin choisi parmi le sodium, le potassium, le rubidium et le césium ou par un ammonium ou phosphonium quaternaire.

8. Procédé selon la revendication 7 **caractérisé par le fait que** ledit acide est sous forme de sel de potassium.

9. Procédé selon l'une des revendications 1 à 8 **caractérisé par le fait que** le dioxyde de soufre est mis en oeuvre sous forme gazeuse ou en solution, dans le solvant organique choisi pour la réaction, à une concentration variant entre 1 et 10 % en poids, de préférence entre 3 et 6 %.

10. Procédé selon l'une des revendications 1 à 9 **caractérisé par le fait que** le solvant organique est un solvant aprotique ayant une constante diélectrique inférieure ou égale à 50 et supérieure ou égale à 5, et est de préférence comprise entre 30 et 40.

11. Procédé selon la revendication 10 **caractérisé par le fait que** le solvant organique polaire présente un nombre donneur supérieur à 10, de préférence compris entre 10 et 30.

12. Procédé selon l'une des revendications 10 et 11 **caractérisé par le fait que** le solvant organique présente un indice accepteur inférieur à 20.

13. Procédé selon l'une des revendications 10 à 12 **caractérisé par le fait que** le solvant organique présente un pKa correspondant à sa première acidité au moins égal à 20, de préférence compris entre 25 et 35.

14. Procédé selon l'une des revendications 10 à 13 **caractérisé par le fait que** le solvant organique est choisi parmi les amides N-disubstitués, y compris les urées tétrasubstituées et les lactames monosubstitués, et les éthers cycliques.

15. Procédé selon la revendication 14 **caractérisé par le fait que** le solvant organique est le N,N-diméthylformamide (DMF), le N,N-diéthylformamide ou le N,N-diméthylacétamide.

16. Procédé selon l'une des revendications 1 à 15 **caractérisé par le fait que** la teneur en protons libérables du milieu réactionnel est au plus égale à 20 %, de préférence au plus égale à 10 %, et plus préférentiellement à 1 % de la concentration molaire en ledit acide fluorocarboxylique.

17. Procédé selon l'une des revendications 1 à 16 **caractérisé par le fait que** la teneur en eau du milieu réactionnel est inférieure à 10 % de la concentration molaire en ledit acide fluorocarboxylique.

18. Procédé selon l'une des revendications 1 à 17 **caractérisé par le fait que** la teneur en les éléments de transition à deux états de valence et en les éléments de la colonne VIII est inférieure à 1000 ppm molaires, de préférence à 10 ppm molaires par rapport audit acide fluorocarboxylique.

19. Procédé selon l'une des revendications 1 à 18 **caractérisé par le fait qu'**il est mis en oeuvre en continu ou en discontinu.

20. Procédé selon l'une des revendications 1 à 19 **caractérisé par le fait que** l'on met en contact l'acide fluorocarboxylique sous forme salifiée, le dioxyde de soufre et le solvant organique et l'on chauffe du mélange réactionnel à une température comprise entre 100°C et 200°C, de préférence entre 120°C et 160°C

21. Procédé selon l'une des revendications 1 à 20 **caractérisé par le fait que** la réaction de sulfination est conduite sous pression atmosphérique.

22. Procédé selon l'une des revendications 1 à 21 **caractérisé par le fait que** le mélange réactionnel résultant comprend deux phases : une phase liquide, où une partie au moins dudit acide et du dioxyde de soufre sont dissous dans ledit solvant et une phase gazeuse contenant essentiellement du dioxyde de soufre et du gaz carbonique formé au cours de la réaction.

23. Procédé selon l'une des revendications 1 à 22 **caractérisé par le fait que** l'on sépare les produits réactionnels alors que le taux de transformation de l'acide fluorocarboxylique est de de 30 à 80 %, de préférence de 40 à 60 %.

24. Procédé selon l'une des revendications 1 à 23 **caractérisé par le fait que** dans une étape suivante, on oxyde le sel d'acide sulfinique précédemment obtenu, par mise en présence de ce dernier avec un réactif d'oxydation, de préférence l'eau oxygénée.

## Patentansprüche

1. Verfahren zur Herstellung eines organischen Fluoroxysulfid-Derivats, bei dem man in Gegenwart eines aprotischen polaren organischen Lösungsmittels
- (i) eine Fluorcarbonsäure der Formel Ea-CF₂-COOH (I), wobei Ea für ein elektronenanziehendes Atom oder eine elektronenanziehende Gruppe steht, die mindestens teilweise durch ein organisches oder anorganisches Kation versalzt ist,
- (ii) und Schwefeldioxid
umsetzt, wobei das Verfahren **dadurch gekennzeichnet ist, dass** das Verhältnis zwischen der Zahl der Mole Schwefeldioxid und der Zahl der Mole Fluorcarbonsäure kleiner als 1, vorzugsweise kleiner als 0,99, ist.

2. Verfahren nach Anspruch 1, **dadurch gekennzeichnet, dass** das Verhältnis zwischen der Zahl der Mole Schwefeldioxid und der Zahl der Mole Fluorcarbonsäure zwischen 0,4 und 0,95, vorzugsweise zwischen 0,7 und 0,9, liegt.

3. Verfahren nach einem der Ansprüche 1 und 2, **dadurch gekennzeichnet, dass** die Fluorcarbonsäure entsprechend der Formel (I), in der die Einheit Ea, die eine elektronenanziehende Wirkung auf das difluorierte Kohlenstoffatom ausübt, vorzugsweise aus den funktionellen Gruppen mit einer Hammett-Konstante σₚ von mindestens 0,1 ausgewählt wird.

4. Verfahren nach einem der Ansprüche 1 bis 3, **dadurch gekennzeichnet, dass** es sich bei der korrespondierenden Fluorcarbonsäure um eine Halogenfluoressigsäure der Formel (Ia):
X-CF₂-COOH (Ia)
handelt, in der:
- X für ein Fluoratom steht.

5. Verfahren nach einem der Ansprüche 1 bis 3, **dadurch gekennzeichnet, dass** es sich bei der korrespondierenden Fluorcarbonsäure um eine Säure der Formel (Ib) :
R-G-CF₂-COOH (Ib)
handelt, in der:
- G für eine funktionelle Gruppe C=O oder S=O steht,
- G für eine Perfluoralkylengruppe -(CF₂)ₙ- steht, wobei n größer gleich 1 ist,
- R für ein Halogenatom, vorzugsweise Chlor oder Fluor, steht,
- R für einen indifferenten organischen oder anorganischen Rest, vorzugsweise einen organischen Rest wie C₆-C₂₀-Aryl, C₁-C₁₅-Alkyl oder C₇-C₁₂-Aralkyl, der gegebenenfalls substituiert ist, steht,
- R auch für einen festen anorganischen oder organischen Träger, wie ein Harz, stehen kann.

6. Verfahren nach Anspruch 5, **dadurch gekennzeichnet, dass** es sich bei der korrespondierenden Fluorcarbonsäure um eine Halogenfluoressigsäure der Formel (Ib) handelt, in der G eine Perfluoralkylengruppe -(CF₂)ₙ- ist, wobei n vorteilhafterweise zwischen 1 und 10, vorzugsweise zwischen 1 und 5, liegt.

7. Verfahren nach einem der Ansprüche 1 bis 6, **dadurch gekennzeichnet, dass** die Säure durch ein aus Natrium, Kalium, Rubidium und Caesium ausgewähltes Alkalimetallkation oder durch ein quaternäres Ammonium oder Phosphonium versalzt ist.

8. Verfahren nach Anspruch 7, **dadurch gekennzeichnet, dass** die Säure in Kaliumsalzform vorliegt.

9. Verfahren nach einem der Ansprüche 1 bis 8, **dadurch gekennzeichnet, dass** das Schwefeldioxid in gasförmiger Form oder in Lösung in dem für die Umsetzung gewählten organischen Lösungsmittel in einer Konzentration zwischen 1 und 10 Gew.-%, vorzugsweise zwischen 3 und 6 Gew.-%, eingesetzt wird.

10. Verfahren nach einem der Ansprüche 1 bis 9, **dadurch gekennzeichnet, dass** es sich bei dem organischen Lösungsmittel um ein aprotisches Lösungsmittel mit einer Dielektrizitätskonstante kleiner gleich 50 und größer gleich 5 und vorzugsweise zwischen 30 und 40 handelt.

11. Verfahren nach Anspruch 10, **dadurch gekennzeichnet, dass** das polare organische Lösungsmittel eine Donorzahl von mehr als 10, vorzugsweise zwischen 10 und 30, aufweist.

12. Verfahren nach einem der Ansprüche 10 und 11, **dadurch gekennzeichnet, dass** das organische Lösungsmittel eine Akzeptorzahl von weniger als 20 aufweist.

13. Verfahren nach einem der Ansprüche 10 bis 12, **dadurch gekennzeichnet, dass** das organische Lösungsmittel einen seiner ersten Acidität entsprechenden pKa-Wert von mindestens 20, vorzugsweise zwischen 25 und 35, aufweist.

14. Verfahren nach einem der Ansprüche 10 bis 13, **dadurch gekennzeichnet, dass** das organische Lösungsmittel aus N-disubstituierten Amiden einschließlich tetrasubstituierter Harnstoffe und monosubstituierter Lactame und cyclischen Ethern ausgewählt wird.

15. Verfahren nach Anspruch 14, **dadurch gekennzeichnet, dass** es sich bei dem organischen Lösungsmittel um N,N-Dimethylformamid (DMF), N,N-Diethylformamid oder N,N-Dimethylacetamid handelt.

16. Verfahren nach einem der Ansprüche 1 bis 15, **dadurch gekennzeichnet, dass** der Gehalt des Reaktionsmediums an freisetzbaren Protonen höchstens 20%, vorzugsweise höchstens 10% und weiter bevorzugt 1% der molaren Konzentration an Fluorcarbonsäure beträgt.

17. Verfahren nach einem der Ansprüche 1 bis 16, **dadurch gekennzeichnet, dass** der Wassergehalt des Reaktionsmediums weniger als 10% der molaren Konzentration an Fluorcarbonsäure beträgt.

18. Verfahren nach einem der Ansprüche 1 bis 17, **dadurch gekennzeichnet, dass** der Gehalt an Übergangselementen mit zwei Wertigkeitsstufen und an Elementen der Gruppe VIII weniger als 1000 Mol-ppm, vorzugsweise 10 Mol-ppm, bezogen auf die Fluorcarbonsäure, beträgt.

19. Verfahren nach einem der Ansprüche 1 bis 18, **dadurch gekennzeichnet, dass** es kontinuierlich oder diskontinuierlich durchgeführt wird.

20. Verfahren nach einem der Ansprüche 1 bis 19, **dadurch gekennzeichnet, dass** man die Fluorcarbonsäure in versalzter Form, das Schwefeldioxid und das organische Lösungsmittel in Berührung bringt und die Reaktionsmischung auf eine Temperatur zwischen 100°C und 200°C, vorzugsweise zwischen 120°C und 160°C, erhitzt.

21. Verfahren nach einem der Ansprüche 1 bis 20, **dadurch gekennzeichnet, dass** die Sulfinierungsreaktion unter Normaldruck durchgeführt wird.

22. Verfahren nach einem der Ansprüche 1 bis 21, **dadurch gekennzeichnet, dass** die resultierende Reaktionsmischung zwei Phasen umfasst: eine flüssige Phase, wobei mindestens ein Teil der Säure und des Schwefeldioxids in dem Lösungsmittel gelöst sind, und eine gasförmige Phase, die im Wesentlichen Schwefeldioxid und im Lauf der Reaktion gebildetes Kohlendioxid enthält.

23. Verfahren nach einem der Ansprüche 1 bis 22, **dadurch gekennzeichnet, dass** man die Reaktionsprodukte trennt, wenn der Umwandlungsgrad der Fluorcarbonsäure 30 bis 80%, vorzugsweise 40 bis 60%, beträgt.

24. Verfahren nach einem der Ansprüche 1 bis 23, **dadurch gekennzeichnet, dass** man in einem nachfolgenden Schritt das oben erhaltene Sulfinsäuresalz durch Zusammenbringen mit einem Oxidationsreagenz, vorzugsweise wässriger Wasserstoffperoxidlösung, oxidiert.

## Claims

1. Process for the preparation of an oxysulfide and fluorinated organic derivative comprising the reaction, in the presence of a polar aprotic organic solvent:
- (i) of a fluorocarboxylic acid of formula Ea - CF₂ - COOH (I),
where Ea represents an electron-withdrawing atom or group, at least partly salified by an organic or inorganic cation,
- (ii) and of sulfur dioxide,
said process being **characterized in that** the ratio of the number of moles of sulfur dioxide to the number of moles of fluorocarboxylic acid is less than 1, preferably less than 0.99.

2. Process according to Claim 1, **characterized in that** the ratio of the number of moles of sulfur dioxide to the number of moles of fluorocarboxylic acid is between 0.4 and 0.95, preferably between 0.7 and 0.9.

3. Process according to either of Claims 1 and 2, **characterized in that** the fluorocarboxylic acid corresponding to the formula (I), in which the entity Ea, which exerts an electron-withdrawing effect on the difluorinated carbon atom, is preferably chosen from the functional groups having a Hammett constant σₚ at least equal to 0.1.

4. Process according to one of Claims 1 to 3, **characterized in that** the corresponding fluorocarboxylic acid is a halofluoroacetic acid of formula (Ia) :
X - CF₂ - COOH (Ia)
in said formula:
- X is a fluorine atom.

5. Process according to one of Claims 1 to 3, **characterized in that** the corresponding fluorocarboxylic acid is an acid of formula (Ib):
R - G - CF₂ - COOH (Ib)
in said formula:
- G represents a C = O or S = O functional group,
- G represents a perfluoroalkylene -(CF₂)ₙ group where n is greater than or equal 1,
- R represents a halogen atom, preferably a chlorine or fluorine atom,
- R represents any inorganic or organic residue, preferably an organic radical, such as C₆-C₂₀ aryl, C₁-C₁₅ alkyl or C₇-C₁₂ aralkyl, which is optionally substituted,
- R can also represent a solid inorganic or organic support, such as a resin.

6. Process according to Claim 5, **characterized in that** the corresponding fluorocarboxylic acid is a halofluoroacetic acid of formula (Ib) in which G represents a perfluoroalkylene group -(CF₂)ₙ-, n advantageously being between 1 and 10, preferably between 1 and 5.

7. Process according to one of Claims 1 to 6, **characterized in that** said acid is salified with an alkali metal cation chosen from sodium, potassium, rubidium and cesium or with a quaternary ammonium or phosphonium.

8. Process according to Claim 7, **characterized in that** said acid is in the potassium salt form.

9. Process according to one of Claims 1 to 8, **characterized in that** sulfur dioxide is employed in the gaseous form or in solution, in the organic solvent chosen for the reaction, at a concentration varying between 1 and 10% by weight, preferably between 3 and 6% by weight.

10. Process according to one of Claims 1 to 9, **characterized in that** the organic solvent is an aprotic solvent having a dielectric constant of less than or equal to 50 and greater than or equal to 5, and preferably between 30 and 40.

11. Process according to Claim 10, **characterized in that** the polar organic solvent exhibits a donor number of greater than 10, preferably of between 10 and 30.

12. Process according to either of Claims 10 and 11, **characterized in that** the organic solvent exhibits an acceptor number of less than 20.

13. Process according to one of Claims 10 to 12, **characterized in that** the organic solvent exhibits a pKa corresponding to its first acidity which is at least equal to 20, preferably between 25 and 35.

14. Process according to one of Claims 10 to 13, **characterized in that** the organic solvent is chosen from N-disubstituted amides, including tetrasubstituted ureas and monosubstituted lactams, and cyclic ethers.

15. Process according to Claim 14, **characterized in that** the organic solvent is N,N-dimethylformamide (DMF), N,N-diethylformamide or N,N-dimethylacetamide.

16. Process according to one of Claims 1 to 15, **characterized in that** the content of releasable protons of the reaction medium is at most equal to 20%, preferably at most equal to 10% and more preferably to 1% of the molar concentration of said fluorocarboxylic acid.

17. Process according to one of Claims 1 to 16, **characterized in that** the water content of the reaction medium is less than 10% of the molar concentration of said fluorocarboxylic acid.

18. Process according to one of Claims 1 to 17, **characterized in that** the content of the transition elements comprising two valence states and of the elements from Group VIII is less than 1000 molar ppm, preferably than 10 molar ppm, with respect to said fluorocarboxylic acid.

19. Process according to one of Claims 1 to 18, **characterized in that** it is carried out continuously or batchwise.

20. Process according to one of Claims 1 to 19, **characterized in that** the fluorocarboxylic acid in the salified form, the sulfur dioxide and the organic solvent are brought into contact and the reaction mixture is heated at a temperature of between 100°C and 200°C, preferably between 120°C and 160°C.

21. Process according to one of Claims 1 to 20, **characterized in that** the sulfination reaction is carried out at atmospheric pressure.

22. Process according to one of Claims 1 to 21, **characterized in that** the resulting reaction mixture comprises two phases: a liquid phase, where a portion at least of said acid and of the sulfur dioxide are dissolved in said solvent, and a gas phase essentially comprising sulfur dioxide and carbon dioxide gas formed during the reaction.

23. Process according to one of Claims 1 to 22, **characterized in that** the reaction products are separated when the degree of conversion of the fluorocarboxylic acid is from 30 to 80%, preferably from 40 to 60%.

24. Process according to one of Claims 1 to 23, **characterized in that**, in a following stage, the salt of sulfinic acid obtained above is oxidized by bringing the latter together with an oxidizing reagent, preferably aqueous hydrogen peroxide solution.
